(19) Europäisches Patentamt / European Patent Office / Office européen des brevets

(11) **EP 4 186 930 A1**

(12) **EUROPEAN PATENT APPLICATION**
published in accordance with Art. 153(4) EPC

(43) Date of publication:
**31.05.2023 Bulletin 2023/22**

(21) Application number: **21846228.1**

(22) Date of filing: **15.07.2021**

(51) International Patent Classification (IPC):
*C08F 20/06* (2006.01)     *A61F 13/53* (2006.01)
*B01J 20/26* (2006.01)     *B01J 20/28* (2006.01)

(52) Cooperative Patent Classification (CPC):
**A61F 13/53; B01J 20/26; B01J 20/28; C08F 20/06**

(86) International application number:
**PCT/JP2021/026700**

(87) International publication number:
**WO 2022/019219 (27.01.2022 Gazette 2022/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.07.2020 JP 2020125175**

(71) Applicant: **Sumitomo Seika Chemicals Co., Ltd.
Kako-gun, Hyogo 675-0145 (JP)**

(72) Inventor: **YAMAMOTO, Tomoe
Himeji-shi, Hyogo 672-8076 (JP)**

(74) Representative: **Mewburn Ellis LLP
Aurora Building
Counterslip
Bristol BS1 6BX (GB)**

(54) **WATER-ABSORBING RESIN COMPOSITION, ABSORBENT, AND ABSORPTIVE ARTICLE**

(57) Provided is a water-absorbing resin composition that is capable of achieving both high liquid diffusion and liquid leakage prevention in an absorbent. The present invention provides a water-absorbing resin composition, wherein: an electromotive force P(mV) of a first-washing washing liquid, obtained as a result of washing the water-absorbing resin composition by means of the washing method indicated below, is equal to or greater than 80 V; and the difference (P-Q) between the electromotive force P(mV) and an electromotive force Q(mV) of a second-washing washing liquid, obtained as a result of washing the water-absorbing resin composition by means of the washing method indicated below, is equal to or greater than 10 mV. (Washing method) A container in which a sieve having a mesh size of 36 μm is attached to the bottom of a cylinder having an inner diameter of 60 mm is prepared. 1 g of the water-absorbing resin composition is evenly dispersed on the sieve in the container. A 200 mL beaker is placed under the sieve. 200 g of a physiological saline solution is rapidly introduced from above the water-absorbing resin composition and the physiological saline solution that has flowed down into the beaker is used as the first-washing washing liquid. Next, a new 200 mL beaker is placed under the sieve. An additional 200 g of the physiological saline solution is rapidly introduced from above the water-absorbing resin composition and the physiological saline solution that has flowed down into the beaker is used as the second-washing washing liquid.

EP 4 186 930 A1

**Description**

TECHNICAL FIELD

The present invention relates to a water-absorbent resin composition, an absorber, and an absorbent article, and more particularly to a water-absorbent resin composition, an absorber, and an absorbent article constituting an absorber suitably used for sanitary materials such as disposable diapers, sanitary napkins, and incontinence pads.

BACKGROUND ART

In recent years, water-absorbent resin particles have been widely used in a field of sanitary materials such as disposable diapers, sanitary napkins, and incontinence pads.

As such water-absorbent resin particles, a partially neutralized acrylic acid polymer crosslinked product is considered to be preferable water-absorbent resin particles because it has an excellent water-absorbent ability, and the acrylic acid as a raw material thereof is easily industrially available, so that it can be produced at a low cost with a constant quality. Further reasons for such preference include its advantages such as its lower possibility of causing decay or deterioration (see, for example, Patent Document 1).

On the other hand, absorbent articles such as disposable diapers, sanitary napkins, or incontinence pads mainly include an absorber that absorbs and holds body fluids such as urine and menstrual blood excreted from a body and is disposed in a central portion, a liquid-permeable top surface sheet (top sheet) disposed on a side in contact with the body, and a liquid-impermeable back surface sheet (back sheet) disposed on an opposite side in contact with the body. In addition, the absorber is usually composed of hydrophilic fibers such as pulp and water-absorbent resin particles.

PRIOR ART DOCUMENT

PATENT DOCUMENT

Patent Document 1: Japanese Patent Laid-open Publication No. H3-227301

SUMMARY OF THE INVENTION

PROBLEMS TO BE SOLVED BY THE INVENTION

In such absorbent articles, the absorption rate of the water-absorbent resin particles contained in the absorber is required to be high. However, if the absorption rate is too high, when liquid is introduced into the absorber, the water-absorbent resin particles quickly absorb the liquid at the place where the liquid is introduced, and the liquid is less likely to spread over the entire absorber, so that a wide range of the absorber may not be effectively utilized. In such a case, there is a problem that the liquid introduced into the absorber for a plurality of times stagnates around the water-absorbent resin particles around the introduced portion and locally reaches saturation, and the liquid that has not been absorbed causes a re-wet phenomenon (that is, a phenomenon that the liquid reverses from the absorber and it is felt as wet when touched with a hand).

As a method of diffusing the liquid over the absorber to suppress the re-wet of the absorber, there is a method of using a water-absorbent resin having a reduced absorption rate. However, the use of the water-absorbent resin having a low absorption rate improves a liquid diffusibility and suppresses the re-wet. On the other hand, due to the diffusibility thereof, when the liquid is introduced for a plurality of times, the liquid may reach an end portion of the absorber, and the liquid may leak from the end portion of the absorber.

Under such circumstances, a main object of the present invention is to provide a water-absorbent resin composition capable of achieving both a high liquid diffusibility and suppression of liquid leakage in an absorber.

MEANS FOR SOLVING THE PROBLEM

The present inventors have conducted intensive studies in order to solve the above problems, and as a method for diffusing a liquid over the entire absorber to suppress the re-wet of the absorber, the present inventors have considered that an acidic compound or the like can be present in the vicinity of the water-absorbent resin composition, an ion concentration in the vicinity of the surface increases to temporarily suppress an affinity with an aqueous liquid, and then the ion concentration in the vicinity of the surface of the water-absorbent resin composition decreases, whereby the liquid diffusibility of the absorber increases and the liquid leakage from the end portion can be suppressed. Specifically, when the liquid is introduced into the absorber for the first time, the acidic compound in the vicinity of the water-absorbent resin particles is dissolved in the liquid, and the ion concentration in the vicinity of the surface is increased, so that the affinity of the water-absorbent resin particles with the liquid is suppressed, and the liquid diffuses over a wide range of the absorber. When the liquid is introduced into the absorber for the second time and more, the acidic compound in the vicinity of the water-absorbent resin particles is diffused together with the liquid, so that the ion concentration in the vicinity of the surface decreases, and the water-absorbent resin particles exhibit a high water absorbency, so that it is considered that the liquid reaches the end portion of the absorber and the liquid leakage from the end portion of the absorber is suitably suppressed. The present invention has been completed through further intensive studies based on such findings.

In other words, the present invention provides an invention with the following configuration.

Item 1. A water-absorbent resin composition, wherein an electromotive force P (mV) of a first-washing washing liquid obtained by washing the water-absorbent resin composition using a washing method below is 80 mV or more; and a difference (P - Q) between an electromotive force Q (mV) of a second-washing washing liquid obtained by further washing the water-absorbent resin composition using a washing method below and the electromotive force P (mV) is 10 mV or more.

(Washing method)

A container in which a sieve mesh having a mesh size of 36 $\mu$m is attached to the bottom of a cylinder with an inner diameter of 60 mm is prepared. The water-absorbent resin composition (1 g) is uniformly sprayed onto the sieve mesh in the container. A beaker of 200 mL is placed under the sieve mesh. 200 g of physiological saline is quickly introduced from above the water-absorbent resin composition, and the physiological saline flowing down into the beaker is used as the first-washing washing liquid. Next, the beaker 200 mL is newly placed under the sieve mesh. Physiological saline (200 g) is further quickly introduced from above the water-absorbent resin composition, and the physiological saline flowing down into the beaker is used as the second-washing washing liquid.

Item 2. The water-absorbent resin composition according to Item 1, wherein the electromotive force Q (mV) of the second-washing washing liquid is 130 mV or less.

Item 3. The water-absorbent resin composition according to Item 1 or 2, wherein the water-absorbent resin composition has a physiological saline absorption amount of 30 to 80 g/g.

Item 4. The water-absorbent resin composition according to any one of items 1 to 3, wherein the water-absorbent resin composition contains water-absorbent resin particles and an acidic compound.

Item 5. The water-absorbent resin composition according to item 4, wherein the water-absorbent resin particles have a median particle size of 200 to 600 $\mu$m, and the acidic compound has a median particle size of 20 to 600 $\mu$m.

Item 6. The water-absorbent resin composition according to item 4 or 5, wherein the acidic compound is contained in an amount of 0.05 to 30 parts by mass with respect to 100 parts by mass of the water-absorbent resin particles.

Item 7. The water-absorbent resin composition according to any one of items 4 to 6, wherein a ratio (T/S) of a median particle size T ($\mu$m) of the water-absorbent resin particles to a median particle size S ($\mu$m) of the acidic compound is 0.1 to 30.

Item 8. The water-absorbent resin composition according to any one of items 4 to 7, wherein a first acid dissociation constant of the acidic compound is 0.1 to 5.0.

Item 9. An absorber including the water-absorbent resin composition according to any one of items 1 to 8.

Item 10. An absorbent article including the absorber according to item 9.

ADVANTAGES OF THE INVENTION

According to the present invention, it is possible to provide a water-absorbent resin composition capable of achieving both a high liquid diffusibility and suppression of liquid leakage in an absorber. Furthermore, according to the present invention, it is also possible to provide an absorber and an absorbent article using the water-absorbent resin composition.

BRIEF DESCRIPTION OF THE DRAWINGS

Fig. 1 shows a schematic view of a testing device used for measuring an electromotive force of a washing liquid for water-absorbent resin composition.

Fig. 2 shows a schematic view of a testing device used for liquid leakage (lateral leakage) test of an absorbent article.

EMBODIMENTS OF THE INVENTION

1. Water-absorbent resin composition

In the water-absorbent resin composition of the present invention, an electromotive force P (mV) of a first-washing washing liquid obtained by washing the water-absorbent resin composition using a washing method below is 80 mV or more. Furthermore, a difference (P - Q) between an electromotive force Q (mV) of the second-washing washing liquid obtained by further washing the water-absorbent resin composition using a washing method below and the electromotive force P (mV) is 10 mV or more.

(Washing method)

A container in which a sieve mesh having a mesh size of 36 μm is attached to the bottom of a cylinder with an inner diameter of 60 mm is prepared. The water-absorbent resin composition (1 g) is uniformly sprayed onto the sieve mesh in the container. A beaker of 200 mL is placed under the sieve mesh. 200 g of physiological saline is quickly introduced from above the water-absorbent resin composition, and the physiological saline flowing down into the beaker is used as the first-washing washing liquid. Next, the beaker 200 mL is newly placed under the sieve mesh. Physiological saline (200 g) is further quickly introduced from above the water-absorbent resin composition, and the physiological saline flowing down into the beaker is used as the second-washing washing liquid. A specific washing method is as described in the examples.

By applying the water-absorbent resin composition of the present invention having such characteristics to an absorber, it is possible to achieve both the high liquid diffusibility and suppression of liquid leakage in the absorber. This mechanism can be considered as follows.

Water absorption behavior of the water-absorbent resin composition is caused by an osmotic pressure generated between the water-absorbent resin composition having a high ion concentration (for example, containing a polyacrylate partially neutralized with sodium) and the liquid to be absorbed (having a relatively low ion concentration). Therefore, as an ion concentration in the liquid to be absorbed increases, the osmotic pressure decreases, a water absorption capacity also decreases, and water absorption characteristics such as an absorption amount and an absorption rate weaken. Furthermore, the ion concentration in the liquid has a positive correlation with the electromotive force, and the higher the electromotive force of the liquid is, the higher the ion concentration is. Therefore, when the electromotive force of the liquid increases, the water absorption characteristic of the water-absorbent resin composition weakens. In the water-absorbent resin composition in which the acidic compound or the like is present in the vicinity of the water-absorbent resin particles, the acidic compound in the vicinity of the water-absorbent resin particles is locally dissolved at the time of first water absorption, the ion concentration in the vicinity of the surface of the water-absorbent resin composition increases, and the water absorption characteristic weakens. As a result, the liquid diffusibility in the absorber is improved, and the re-wet of liquid in the absorber is suppressed. Furthermore, the ion concentration in the vicinity of the surface of the water-absorbent resin composition decreases as the acidic compound diffuses in the absorber or is absorbed by the water-absorbent resin particles. Accordingly, in the water-absorbent resin composition during second or third liquid absorption, a weakening of water absorption characteristic is suppressed. Therefore, liquid absorption is superior to liquid diffusion, and liquid leakage from the end portion of the absorber when liquid is introduced for a plurality of times is suppressed. Hereinafter, the water-absorbent resin composition of the present invention will be described in detail.

From a viewpoint of more suitably exhibiting an effect of the present invention, the electromotive force P (mV) of the first-washing washing liquid is 80 mV or more, preferably 90 mV or more, more preferably 100 mV or more, and still more preferably 120 mV or more. In addition, the electromotive force P (mV) is preferably 230 mV or less, more preferably 200 mV or less, still more preferably 190 mV or less, and further more preferably 180 mV or less. The range of the electromotive force P (mV) is preferably 80 to 230 mV, more preferably 90 to 200 mV, still more preferably 100 to 190 mV, and further more preferably 120 to 180 mV.

Furthermore, from the viewpoint of more suitably exhibiting the effect of the present invention, the electromotive force Q (mV) of the second-washing washing liquid is preferably 20 mV or more, more preferably 30 mV or more, still more preferably 40 mV or more, and further more preferably 50 mV or more. In addition, the electromotive force Q (mV) is preferably 145 mV or less, more preferably 125 mV or less, still more preferably 100 mV or less, and further more preferably 90 mV or less. The range of the electromotive force Q (mV) is preferably 20 to 145 mV, more preferably 30 to 125 mV, still more preferably 40 to 100 mV, and further more preferably 50 to 90 mV.

Also, from the viewpoint of more suitably exhibiting the effect of the present invention, the difference (P - Q) between the electromotive force Q (mV) and the electromotive force P (mV) is 10 mV or more, preferably 25 mV or more, more preferably 35 mV or more, and still more preferably 50 mV or more. In addition, the difference (P - Q) is preferably 200 mV or less, more preferably 150 mV or less, still more preferably 130 mV or less, and further more preferably 110 mV or less. The range of the difference (P - Q) is preferably 10 to 200 mV, more preferably 25 to 150 mV, still more preferably 35 to 130 mV, and further more preferably 50 to 110 mV.

The physiological saline absorption amount of the water-absorbent resin composition of the present invention is preferably 30 g/g or more, 35 g/g or more, 40 g/g or more, 45 g/g or more, 50 g/g or more, or 55 g/g or more from a viewpoint of easily enhancing liquid leakage performance in the absorbent article. In addition, the absorption amount is preferably 80 g/g or less, 75 g/g or less, 70 g/g or less, 65 g/g or less, 60 g/g or less, or 63 g/g or less from a viewpoint of easily improving the liquid diffusibility in the absorbent article. From these viewpoints, a water retention amount is preferably 30 to 80 g/g, and more preferably 45 to 65 g/g.

From the viewpoint of setting the electromotive force P (mV), the difference (P - Q) between the electromotive force Q (mV) and the electromotive force P (mV), and even the electromotive force Q (mV) of the water-absorbent resin composition of the present invention to the predetermined values, and more suitably exhibiting the effect of the present invention, the water-absorbent resin composition preferably contains water-absorbent resin particles and the acidic compound. More specifically, in the water-absorbent resin composition of the present invention, the acidic compound is preferably present on at least one part of the surface of the water-absorbent resin particles. For example, by mixing the water-absorbent resin particles and the acidic compound in a solid phase state, the acidic compound can be present on the surface of the water-absorbent resin composition to such an extent that the effect of the present invention can be exhibited.

As described above, the ion concentration in the liquid has a positive correlation with the electromotive force, and the higher the electromotive force of the liquid is, the higher the ion concentration is. In the water-absorbent resin composition wherein the acidic compound is present in the vicinity of the water-absorbent resin particles, the acidic compound on the surface is locally dissolved at the time of initial water absorption, the ion concentration in the vicinity of the surface of the water-absorbent resin composition increases, and the electromotive force of the liquid increases. When the water-absorbent resin composition of the present invention contains the acidic compound, from the viewpoint of setting the electromotive force P (mV), the difference (P - Q) between the electromotive force Q (mV) and the electromotive force P (mV), and further the electromotive force Q (mV) of the water-absorbent resin composition of the present invention to the predetermined values, and more suitably exhibiting the effect of the present invention, the acidic compound is preferably a solid in a normal temperature (25°C) and a normal pressure (1 atm) environment. From the same viewpoint, the acidic compound is preferably water-soluble. Additionally, in the present invention, the term "water-soluble" means that water has a solubility of 0.5 mass% or more at the normal temperature and the normal pressure.

From the same viewpoint, the first acid dissociation constant of the acidic compound is preferably 0.1 to 5.0, more preferably 0.5 to 4.5, and still more preferably 1.0 to 4.0. Furthermore, when the acidic compound has a second acid dissociation constant, the second acid dissociation constant is preferably 2.0 to 7.0, more preferably 2.5 to 6.5, and still more preferably 3.0 to 6.0. In addition, when the acidic compound has a third acid dissociation constant, the third acid dissociation constant is preferably 3.0 to 7.0, more preferably 3.5 to 6.5, and still more preferably 4.0 to 6.0.

From the same viewpoint, the median particle size of the acidic compound is preferably about 20 to 600 $\mu$m, more preferably about 30 to 500 $\mu$m, and still more preferably about 40 to 400 $\mu$m. The median particle size of the acidic compound can be measured using JIS standard sieves, and is specifically a value measured using a method described in Examples.

From the same viewpoint, the ratio (T/S) of the median particle size T ($\mu$m) of the water-absorbent resin particles to the median particle size S ($\mu$m) of the acidic compound is preferably 0.1 to 30, more preferably 0.5 to 20, still more preferably 0.8 to 15, and further more preferably 1.0 to 10.

The acidic compound is preferably an organic acid, and among organic acids, tartaric acid, citric acid, malic acid, fumaric acid, sorbic acid, maleic acid, salicylic acid, succinic acid, adipic acid, glutaric acid, glycolic acid, phthalic acid, mandelic acid, and benzoic acid are particularly preferable. Furthermore, tartaric acid, citric acid, malic acid, and fumaric acid are more preferable. The acidic compound contained in the water-absorbent resin composition of the present invention may be one kind or two or more kinds.

From the viewpoint of more suitably exerting the effect of the present invention, a content of the acidic compound in the water-absorbent resin composition of the present invention is preferably 0.05 to 30 parts by mass, more preferably 0.1 to 20 parts by mass, still more preferably 0.5 to 15 parts by mass, and further more preferably 1 to 10 parts by mass, based on 100 parts by mass of the water-absorbent resin particles.

Next, the water-absorbent resin particles contained in the water-absorbent resin composition of the present invention will be described in detail.

(Water-absorbent resin particles)

The water-absorbent resin particles contained in the water-absorbent resin composition of the present invention are formed by crosslinking a polymer of a water-soluble ethylenically unsaturated monomer, that is, a crosslinked polymer with a structural unit derived from a water-soluble ethylenically unsaturated monomer.

Water-absorbent resins are usually in the form of particles. The water-absorbent resin particles preferably have a median particle size of 200 to 600 μm, more preferably 200 to 500 μm, and still more preferably 250 to 450 μm. In other words, regarding the water-absorbent resin composition of the present invention, the median particle size is preferably 200 to 600 μm, more preferably 200 to 500 μm, still more preferably 250 to 450 μm, and further more preferably 300 to 425 μm.

Furthermore, the water-absorbent resin particles may be in a form of fine particles (primary particles) aggregated together (secondary particles) as well as each consisting of a single particle. Examples of shapes of the primary particles include a substantial spherical shape, an indefinite crushed shape, a plate shape, and the like. Examples of the primary particles produced by a reversed-phase suspension polymerization include substantial spherical single particles with a smooth surface shape such as a perfect spherical shape and an elliptical spherical shape, and the primary particles with such a shape have high fluidity as a powder due to the smooth surface shape, and are less likely to be broken specifically when subjected to an impact because the aggregated particles are easily densely packed and become water-absorbent resin particles with high particle strength.

The median particle size of the water-absorbent resin particles can be measured using JIS standard sieves. Specifically, the median particle size is a value measured by a method described in Examples.

As a polymerization method of the water-soluble ethylenically unsaturated monomer, an aqueous solution polymerization method, an emulsion polymerization method, a reversed-phase suspension polymerization method, and the like, which are representative polymerization methods, are used. In the aqueous solution polymerization method, polymerization is performed by heating a water-soluble ethylenically unsaturated monomer solution while stirring the aqueous solution as necessary. Furthermore, in the reversed-phase suspension polymerization method, polymerization is performed by heating the water-soluble ethylenically unsaturated monomer in a hydrocarbon dispersion medium under stirring. The reversed-phase suspension polymerization method is preferably used from a viewpoint of enabling precise polymerization reaction control and wide particle size control.

An example of the method for producing the water-absorbent resin particles will be described below.

Specific examples of the method for producing the water-absorbent resin particles include a method for producing water-absorbent resin particles by subjecting the water-soluble ethylenically unsaturated monomer to the reversed-phase suspension polymerization in the hydrocarbon dispersion medium, the method including steps of: performing polymerization in the presence of a radical polymerization initiator; and post-crosslinking the hydrous gel-like material obtained by polymerization in the presence of a post-crosslinking agent. Furthermore, in the method for producing water-absorbent resin particles of the present invention, an internal crosslinking agent may be added to the water-soluble ethylenically unsaturated monomer as necessary to form the hydrous gel-like material with an internally crosslinked structure.

<Polymerization step>

[Water-soluble ethylenically unsaturated monomer]

Examples of the water-soluble ethylenically unsaturated monomer include (meth)acrylic acid (in the present description, "acrylic" and "methacrylic" are collectively referred to as "(meth)acrylic"; and the same applies hereinafter) and salts thereof; 2-(meth)acrylamide-2 methylpropane sulfonic acid and salts thereof; nonionic monomers such as (meth)acrylamide, N,N-dimethyl(meth)acrylamide, 2-hydroxyethyl(meth)acrylate, N-methylol(meth)acrylamide, and polyethylene glycol mono(meth)acrylate; amino group-containing unsaturated monomers such as N,N-diethylaminoethyl(meth)acrylate, N,N-diethylaminopropyl(meth)acrylate, and diethylaminopropyl(meth)acrylamide, and quaternary products thereof; and the like. Among these water-soluble ethylenically unsaturated monomers, the (meth)acrylic acid or salts thereof, (meth)acrylamide, and N,N-dimethylacrylamide are preferable, and the (meth)acrylic acid and salts thereof are more preferable from a viewpoint of industrial availability and the like. In addition, these water-soluble ethylenically unsaturated monomers may be used alone or in a combination of two or more kinds thereof.

Among them, the acrylic acid and salts thereof are widely used as raw materials of water-absorbent resins, and such acrylic acid and/or salts thereof may be copolymerized with the other water-soluble ethylenically unsaturated monomers mentioned above and used. In this case, acrylic acid and/or salts thereof is preferably used as a main water-soluble ethylenically unsaturated monomer in an amount of 70 to 100 mol% with respect to a total amount of water-soluble ethylenically unsaturated monomers.

The water-soluble ethylenically unsaturated monomer is preferably dispersed in the hydrocarbon dispersion medium in a state of an aqueous solution and subjected to the reversed-phase suspension polymerization. When the water-soluble ethylenically unsaturated monomer is an aqueous solution, a dispersion efficiency in the hydrocarbon dispersion medium can be increased. The concentration of the water-soluble ethylenically unsaturated monomer in the aqueous solution is preferably in a range of 20 mass% to a saturated concentration or less. Furthermore, the concentration of the water-soluble ethylenically unsaturated monomer is more preferably 55 mass% or less, still more preferably 50 mass% or less, and still more preferably 45 mass% or less. On the other hand, the concentration of the water-soluble ethylenically unsaturated monomer is more preferably 25 mass% or more, still more preferably 28 mass% or more, and further more preferably 30 mass% or more.

When the water-soluble ethylenically unsaturated monomer has an acid group such as (meth)acrylic acid or 2-(meth)acrylamide-2-methylpropane sulfonic acid, the acid group may be neutralized in advance with an alkaline neutralizing agent as necessary. Examples of such alkaline neutralizing agents include alkali metal salts such as sodium hydroxide, sodium carbonate, sodium hydrogen carbonate, potassium hydroxide, and potassium carbonate; ammonia and the like. In addition, these alkaline neutralizing agents may be used in a form of an aqueous solution in order to simplify neutralization operations. In addition, the alkaline neutralizing agents described above may be used alone, or may be used in a combination of two or more kinds thereof.

A neutralization degree of the water-soluble ethylenically unsaturated monomer by the alkaline neutralizing agents is preferably 10 to 100 mol%, more preferably 30 to 90 mol%, still more preferably 40 to 85 mol%, and further more preferably 50 to 80 mol% as the neutralization degree with respect to all acid groups of the water-soluble ethylenically unsaturated monomer.

[Radical polymerization initiator]

Examples of radical polymerization initiators added to the polymerization step include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; azo compounds such as 2,2'-azobis(2-amidinopropane)dihydrochloride, 2,2'-azobis[2-(N-phenylamidino)propane]dihydrochloride, 2,2'-azobis[2-(N-allylamidino)propane]dihydrochloride, 2,2'-azobis{2-[1-(2-hydroxyethyl)-2-imidazolin-2-yl]propane }dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid); and the like. Among the radical polymerization initiators, the potassium persulfate, the ammonium persulfate, the sodium persulfate, and 2,2'-azobis(2-amidinopropane)dihydrochloride are preferable from a viewpoint of easy availability and easy handling. The radical polymerization initiators may be used alone or in combination of two or more. Furthermore, the radical polymerization initiators can also be used as redox polymerization initiators in combination with reducing agents such as sodium sulfite, sodium bisulfite, ferrous sulfate, or L-ascorbic acid.

An amount of the radical polymerization initiators used is, for example, 0.00005 to 0.01 mol relative to 1 mol of the water-soluble ethylenically unsaturated monomer. By satisfying such an amount to be used, an occurrence of a rapid polymerization reaction can be avoided, and the polymerization reaction can be completed within an appropriate time.

[Internal crosslinking agent]

Examples of internal crosslinking agents include those capable of crosslinking a polymer of the water-soluble ethylenically unsaturated monomer to be used, and for example, (poly)ethylene glycol ["(poly)" in (poly)ethylene glycol means a case where there is or is not a prefix of "poly"; and the same applies hereinafter]; unsaturated polyesters obtained by reacting polyols such as diols and triols like (poly)propylene glycol, 1,4-butanediol, trimethylolpropane, and (poly)glycerin with unsaturated acids such as (meth)acrylic acid, maleic acid, and fumaric acid; bisacrylamides such as N,N-methylenebisacrylamide; di(meth)acrylic acid esters or tri(meth)acrylic acid esters obtained by reacting polyepoxide with (meth)acrylic acid; di(meth)acrylic acid carbamyl esters obtained by reacting polyisocyanate like tolylene diisocyanate and hexamethylene diisocyanate with hydroxyethyl(meth)acrylate; compounds with two or more polymerizable unsaturated groups, such as allylated starch, allylated cellulose, diallyl phthalate, N,N',N"-triallyl isocyanurate, and divinylbenzene; diglycidyl compounds such as (poly)ethylene glycol diglycidyl ether, (poly)propylene glycol diglycidyl ether, and (poly) glycerin diglycidyl ether, and polyglycidyl compounds such as triglycidyl compounds; epihalohydrin compounds such as epichlorohydrin, epibromhydrin, and $\alpha$-methylepichlorohydrin; compounds with two or more reactive functional groups such as isocyanate compounds like 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; and the like. Among the internal crosslinking agents, unsaturated polyesters or polyglycidyl compounds are preferably used, diglycidyl ether compounds are more preferably used, and (poly)ethylene glycol diglycidyl ether, (poly)propylene glycol diglycidyl ether, and (poly)glycerin diglycidyl ether are preferably used. The internal crosslinking agents may be used alone, or may be used in a combination of two or more kinds thereof.

An amount of the internal crosslinking agents to be used is preferably 0.000001 to 0.02 mol, more preferably 0.00001 to 0.01 mol, still more preferably 0.00001 to 0.005 mol, and further more preferably 0.00005 to 0.002 mol, relative to 1 mol of the water-soluble ethylenically unsaturated monomer.

[Hydrocarbon dispersion medium]

Examples of hydrocarbon dispersion mediums include aliphatic hydrocarbons with 6 to 8 carbon atoms such as n-hexane, n-heptane, 2-methylhexane, 3-methylhexane, 2,3-dimethylpentane, 3-ethylpentane, and n-octane; alicyclic hydrocarbon such as cyclohexane, methylcyclohexane, cyclopentane, methylcyclopentane, trans-1,2-dimethylcyclopentane, cis-1,3-dimethylcyclopentane, and trans-1,3-dimethylcyclopentane; aromatic hydrocarbons such as benzene, toluene, and xylene; and the like. Among the hydrocarbon dispersion mediums, n-hexane, n-heptane, and cyclohexane are particularly preferably used because these compounds are industrially easily available, have stable quality, and are inexpensive. The hydrocarbon dispersion mediums may be used alone or in a combination of two or more kinds thereof. In addition, as an example of a mixture of the hydrocarbon dispersion mediums, a suitable result can be obtained by using a commercially available product such as exol heptane (manufactured by Exxon Mobil Corporation: containing 75 to 85 mass% of hydrocarbon of heptane and isomers thereof).

An amount of the hydrocarbon dispersion mediums to be used is preferably 100 to 1500 parts by mass, and more preferably 200 to 1400 parts by mass relative to 100 parts by mass of a first-stage water-soluble ethylenically unsaturated monomer from a viewpoint of uniformly dispersing the water-soluble ethylenically unsaturated monomer and easily controlling a polymerization temperature. In addition, as will be described later, the reversed-phase suspension polymerization is performed in one stage (single stage) or two or more stages, and the first-stage polymerization described above means a first-stage polymerization reaction in a single-stage polymerization or a multistage polymerization (the same applies hereinafter).

[Dispersion stabilizer]

(Surfactant)

In the reversed-phase suspension polymerization, a dispersion stabilizer can also be used in order to improve a dispersion stability of the water-soluble ethylenically unsaturated monomer in the hydrocarbon dispersion medium. As the dispersion stabilizer, a surfactant can be used.

As surfactants, for example, sucrose fatty acid ester, polyglycerol fatty acid ester, sorbitan fatty acid ester, polyoxyethylene sorbitan fatty acid ester, polyoxyethylene glycerin fatty acid ester, sorbitol fatty acid ester, polyoxyethylene sorbitol fatty acid ester, polyoxyethylene alkyl ether, polyoxyethylene alkyl phenyl ether, polyoxyethylene castor oil, polyoxyethylene hydrogenated castor oil, alkylallyl formaldehyde condensed polyoxyethylene ether, a polyoxyethylene polyoxypropylene block copolymer, polyoxyethylene polyoxypropyl alkyl ether, polyethylene glycol fatty acid ester, alkyl glucoside, N-alkyl gluconamide, polyoxyethylene fatty acid amide, polyoxyethylene alkylamine, phosphoric acid ester of polyoxyethylene alkyl ether, phosphoric acid ester of polyoxyethylene alkyl allyl ether, and the like can be used. Among the surfactants, it is particularly preferable to use sorbitan fatty acid ester, polyglycerin fatty acid ester, or sucrose fatty acid ester from a viewpoint of dispersion stability of the monomer. The surfactants may be used alone or in a combination of two or more kinds thereof.

An amount of the surfactant to be used is preferably 0.1 to 30 parts by mass, and more preferably 0.3 to 20 parts by mass, relative to 100 parts by mass of the first-stage water-soluble ethylenically unsaturated monomer.

(Polymeric dispersant)

As the dispersion stabilizer used in the reversed-phase suspension polymerization, a polymeric dispersant may be used together with the surfactant described above.

Examples of the polymeric dispersant include maleic anhydride-modified polyethylene, maleic anhydride-modified polypropylene, maleic anhydride-modified ethylene-propylene copolymer, maleic anhydride-modified EPDM (ethylene-propylenediene terpolymer), maleic anhydride-modified polybutadiene, maleic anhydride-ethylene copolymer, maleic anhydride-propylene copolymer, maleic anhydride-ethylene-propylene copolymer, maleic anhydride-butadiene copolymer, polyethylene, polypropylene, ethylene-propylene copolymer, oxidized polyethylene, oxidized polypropylene, oxidized ethylene-propylene copolymer, ethylene-acrylic acid copolymer, ethyl cellulose, ethyl hydroxyethyl cellulose, and the like. Among the polymeric dispersants, maleic anhydride-modified polyethylene, maleic anhydride-modified polypropylene, maleic anhydride-modified ethylene-propylene copolymer, maleic anhydride-ethylene copolymer, maleic anhydride-propylene copolymer, maleic anhydride-ethylene-propylene copolymer, polyethylene, polypropylene, ethylene-propylene copolymer, oxidized polyethylene, oxidized polypropylene, and oxidized ethylene-propylene copolymer are particularly preferably used from a viewpoint of dispersion stability of monomers. These polymeric dispersants may be used alone or in a combination of two or more kinds thereof.

An amount of the polymeric dispersants to be used is preferably 0.1 to 30 parts by mass, and more preferably 0.3 to 20 parts by mass relative to 100 parts by mass of the first-stage water-soluble ethylenically unsaturated monomer.

[Other components]

In a method for producing water-absorbent resin particles, if desired, other components may be added to an aqueous solution containing the water-soluble ethylenically unsaturated monomer to perform the reversed-phase suspension polymerization. As other components, various additives such as a thickener and a chain transfer agent can be added.

As an example, the reversed-phase suspension polymerization can be performed by adding a thickener to an aqueous solution containing the water-soluble ethylenically unsaturated monomer. By thus adding a thickener to adjust a viscosity of the aqueous solution, it is possible to control a median particle size obtained in the reversed-phase suspension polymerization.

As a thickener, for example, hydroxyethyl cellulose, hydroxypropyl cellulose, methyl cellulose, carboxymethyl cellulose, polyacrylic acid, a (partial) neutralized polyacrylic acid, polyethylene glycol, polyacrylamide, polyethyleneimine, dextrin, sodium alginate, polyvinyl alcohol, polyvinyl pyrrolidone, polyethylene oxide, and the like can be used. In addition, when a stirring rate during polymerization is the same, primary particles and/or secondary particles of the obtained particles tend to be larger as a viscosity of the water-soluble ethylenically unsaturated monomer aqueous solution becomes higher.

[Reversed-phase suspension polymerization]

In performing the reversed-phase suspension polymerization, for example, an aqueous monomer solution containing the water-soluble ethylenically unsaturated monomer is dispersed in the hydrocarbon dispersion medium in the presence of the dispersion stabilizer. At this time, before a polymerization reaction is started, the addition timing of the dispersion stabilizer (the surfactant or the polymeric dispersant) may be either before or after the addition of the aqueous monomer solution.

Among them, from a viewpoint of easily reducing the amount of the hydrocarbon dispersion medium remaining in the obtained water-absorbent resin particles, it is preferable to disperse the aqueous monomer solution in the hydrocarbon dispersion medium in which the polymeric dispersant is dispersed, and then further disperse the surfactant to perform polymerization.

Such reversed-phase suspension polymerization can be performed in one stage or two or more stages. In addition, from a viewpoint of enhancing productivity, it is preferable to perform in two to three stages.

When the reversed-phase suspension polymerization is performed in two or more stages, after a first-stage reversed-phase suspension polymerization is performed, the water-soluble ethylenically unsaturated monomer may be added to and mixed with the reaction mixture obtained in the first-stage polymerization reaction, and a second-stage or later reversed-phase suspension polymerization may be performed in a same manner as the first-stage polymerization. In the reversed-phase suspension polymerization in each of the second and subsequent stages, in addition to the water-soluble ethylenically unsaturated monomer, the radical polymerization initiator is preferably added within a range of a molar ratio of each component to the water-soluble ethylenically unsaturated monomer described above based on the amount of the water-soluble ethylenically unsaturated monomer added in the reversed-phase suspension polymerization in each of the second and subsequent stages to perform the reversed-phase suspension polymerization. In addition, in the second and subsequent stages of polymerization, an internal crosslinking agent may be added to the water-soluble ethylenically unsaturated monomer as necessary.

A reaction temperature of the polymerization reaction is preferably 20 to 110°C and more preferably 40 to 90°C from a viewpoint of enhancing economic efficiency by rapidly progressing the polymerization and shortening polymerization time, and easily removing heat of polymerization to smoothly perform the reaction.

<Post-crosslinking step>

Next, water-absorbent resin particles of the present invention are obtained by adding a post-crosslinking agent to a hydrous gel-like material with an internally crosslinked structure obtained by polymerizing the water-soluble ethylenically unsaturated monomer, and crosslinking the hydrous gel-like material (a post-crosslinking reaction). The post-crosslinking reaction is preferably performed in the presence of the post-crosslinking agent after polymerization of the water-soluble ethylenically unsaturated monomer. As described above, by subjecting the hydrous gel-like material with an internally-crosslinked structure to the post-crosslinking reaction after polymerization, it is possible to obtain water-absorbent resin particles in which the crosslinking density in the vicinity of a surface of the water-absorbent resin particles is increased and various performances such as water absorption capacity under load are improved.

Examples of the post-crosslinking agent include compounds with two or more reactive functional groups. For example, polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromhydrin, and α-methylepichlorohydrin; isocyanate compounds such as 2,4-tolylene diisocyanate and hexamethylene diisocyanate; oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds such as 1,2-ethylene bisoxazoline; carbonate compounds such as ethylene carbonate; hydroxyalkylamide compounds such as bis[N,N-di(β-hydroxyethyl)]adipamide; and the like. Among the post-crosslinking agent, polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether are preferable. The post-crosslinking agent may be used alone, or may be used in a combination of two or more kinds thereof.

An amount of the post-crosslinking agent to be used is preferably 0.00001 to 0.01 mol, more preferably 0.00005 to 0.005 mol, and still more preferably 0.0001 to 0.002 mol, relative to 1 mol of a total amount of the water-soluble ethylenically unsaturated monomers used for polymerization.

As a method for adding the post-crosslinking agent, the post-crosslinking agent may be added as it is or as an aqueous solution, or may be added as a solution using a hydrophilic organic solvent as a solvent as necessary. Examples of the hydrophilic organic solvents include lower alcohols such as methyl alcohol, ethyl alcohol, n-propyl alcohol, and isopropyl alcohol; ketones such as acetone and methyl ethyl ketone; ethers such as diethyl ether, dioxane, and tetrahydrofuran; amides such as N,N-dimethylformamide; sulfoxides such as dimethyl sulfoxide; and the like. The hydrophilic organic solvent may be used alone, or may be used in a combination of two or more kinds thereof, or as a mixed solvent with water.

The addition timing of the post-crosslinking agent may be after almost all the polymerization reaction of the water-soluble ethylenically unsaturated monomer is completed, and the post-crosslinking agent is preferably added in the presence of moisture in a range of 1 to 400 parts by mass, more preferably in the presence of moisture in a range of 5 to 200 parts by mass, still more preferably in the presence of moisture in a range of 10 to 100 parts by mass, and further more preferably in the presence of moisture in a range of 20 to 60 parts by mass relative to 100 parts by mass of the water-soluble ethylenically unsaturated monomer. Furthermore, an amount of moisture means a total amount of moisture contained in a reaction system and moisture used as necessary when the post-crosslinking agent is added.

The reaction temperature in the post-crosslinking reaction is preferably 50 to 250°C, more preferably 60 to 180°C, still more preferably 60 to 140°C, and further more preferably 70 to 120°C. Furthermore, the reaction time of the post-crosslinking reaction is preferably 1 to 300 minutes, and more preferably 5 to 200 minutes.

<Drying step>

A method may include a drying step of removing water, the hydrocarbon dispersion medium, and the like by distillation by externally applying an energy such as heat after performing the reversed-phase suspension polymerization described above. When dehydration is performed from the hydrous gel after the reversed-phase suspension polymerization, the system in which the hydrous gel is dispersed in the hydrocarbon dispersion medium is heated, so that water and the hydrocarbon dispersion medium are temporarily distilled off to the outside of the system by azeotropic distillation. At this time, when only the distilled hydrocarbon dispersion medium is returned into the system, continuous azeotropic distillation becomes possible. In that case, since a temperature in the system during drying is maintained at an azeotropic temperature with the hydrocarbon dispersion medium or lower, it is preferable from a viewpoint that the resin is hardly deteriorated. Subsequently, water and the hydrocarbon dispersion medium are distilled off to obtain water-absorbent resin particles. Various performances of the water-absorbent resin particles to be obtained can be controlled by controlling the treatment conditions in the drying step after the polymerization to adjust an amount of water to be removed.

In the drying step, drying treatment by distillation may be performed under a normal pressure or under a reduced pressure. In addition, from a viewpoint of enhancing a drying efficiency, the drying may be performed under a flow of nitrogen or the like. When the drying treatment is performed under the normal pressure, a drying temperature is preferably 70 to 250°C, more preferably 80 to 180°C, still more preferably 80 to 140°C, and further more preferably 90 to 130°C. When the drying treatment is performed under the reduced pressure, a drying temperature is preferably 40 to 160°C, and more preferably 50 to 110°C.

When the post-crosslinking step with the post-crosslinking agent is performed after the polymerization of the monomer is performed by reversed-phase suspension polymerization, the drying step by distillation described above is performed after the completion of the post-crosslinking step. Alternatively, the post-crosslinking step and the drying step may be performed simultaneously.

The water-absorbent resin composition of the present invention may contain an additive according to the purpose. Examples of such additives include inorganic powders, surfactants, oxidants, reducing agents, metal chelating agents, radical chain inhibitors, antioxidants, and antibacterial agents. For example, a fluidity of the water-absorbent resin composition can be further improved by adding 0.05 to 5 parts by mass of amorphous silica as an inorganic powder to 100 parts by mass of the water-absorbent resin particles.

In addition, in the water-absorbent resin composition of the present invention, a content of the water-absorbent resin particles (excluding additives) is preferably 70 mass% or more, more preferably 80 mass% or more, and still more preferably 90 mass% or more.

The water-absorbent resin composition of the present invention can be suitably produced, for example, by a method including a step of mixing water-absorbent resin particles, which are crosslinked polymers of the water-soluble ethylenically unsaturated monomer, the internal crosslinking agent, and the post-crosslinking agent described above, with the acidic compound. A temperature of the mixing step may be 10 to 100°C. A temperature in the mixing step is preferably 15 to 50°C and a relative humidity is preferably 40 to 75%. For example, by mixing the water-absorbent resin particles and the acidic compound in a solid phase state, the acidic compound can be present on the surface of the water-absorbent resin particles to such an extent that the effect of the present invention can be exhibited. In addition, the water-absorbent resin composition of the present invention may be prepared by mixing the acidic compound with water-absorbent resin particles in a state of being dissolved or dispersed in a liquid medium such as an aqueous liquid.

2. Absorber, absorbent article

The water-absorbent resin composition of the present invention constitutes an absorber used for sanitary materials such as sanitary products and disposable diapers, and is suitably used for an absorbent article including the absorber.

Here, the absorber using the water-absorbent resin composition of the present invention contains the water-absorbent resin composition of the present invention. The absorber may further include hydrophilic fibers. Examples of configurations of the absorber include a sheet-like structure in which the water-absorbent resin composition is fixed on a nonwoven fabric or between a plurality of nonwoven fabrics, a mixed dispersion obtained by mixing the water-absorbent resin composition and hydrophilic fibers so as to have a uniform composition, a sandwich structure in which the water-absorbent resin composition is sandwiched between layered hydrophilic fibers, a structure in which the water-absorbent resin composition and the hydrophilic fibers are wrapped with tissue, and the like. In addition, the absorber may contain other components, for example, an adhesive binder such as a heat-sealable synthetic fiber, a hot melt adhesive, or an adhesive emulsion for enhancing a shape retention of the absorber.

A content of the water-absorbent resin composition in the absorber is preferably 5 to 100 mass%, more preferably 10 to 95 mass%, still more preferably 20 to 90 mass%, and further more preferably 30 to 80 mass%.

Examples of the hydrophilic fibers include cellulose fibers such as fluff pulp obtained from wood, mechanical pulp, chemical pulp, and semi-chemical pulp; artificial cellulose fibers such as rayon and acetate; and fibers made of synthetic resins such as hydrophilized polyamide, polyester, and polyolefin; and the like. An average fiber length of the hydrophilic fibers is usually 0.1 to 10 mm or may be 0.5 to 5 mm.

The absorber using the water-absorbent resin composition of the present invention can be held between a liquid-permeable sheet (a top sheet) through which a liquid can pass and a liquid-impermeable sheet (a back sheet) through which a liquid cannot pass to form the absorbent article of the present invention. The liquid-permeable sheet is disposed on a side in contact with the body, and the liquid-impermeable sheet is disposed on the opposite side in contact with the body.

Examples of the liquid permeable sheet include nonwoven fabrics such as an air-through type, a spunbond type, a chemical bond type, and a needle punch type made of fibers such as polyethylene, polypropylene, and polyester, porous synthetic resin sheets, and the like. Examples of the liquid-impermeable sheet include synthetic resin films made of resins such as polyethylene, polypropylene, and polyvinyl chloride, and the like.

EXAMPLES

Hereinafter, the present invention will be described in detail with reference to the examples and comparative examples. However, the present invention is not limited to the examples.

The water-absorbent resin compositions obtained in the following examples and comparative examples were evaluated in various tests below. In addition, unless otherwise specified, the measurement was performed under an environment of a temperature of 25 ± 2°C and a humidity of 50 ± 10%. Hereinafter, each evaluation test method will be described.

<Electromotive force of washing liquid for water-absorbent resin composition>

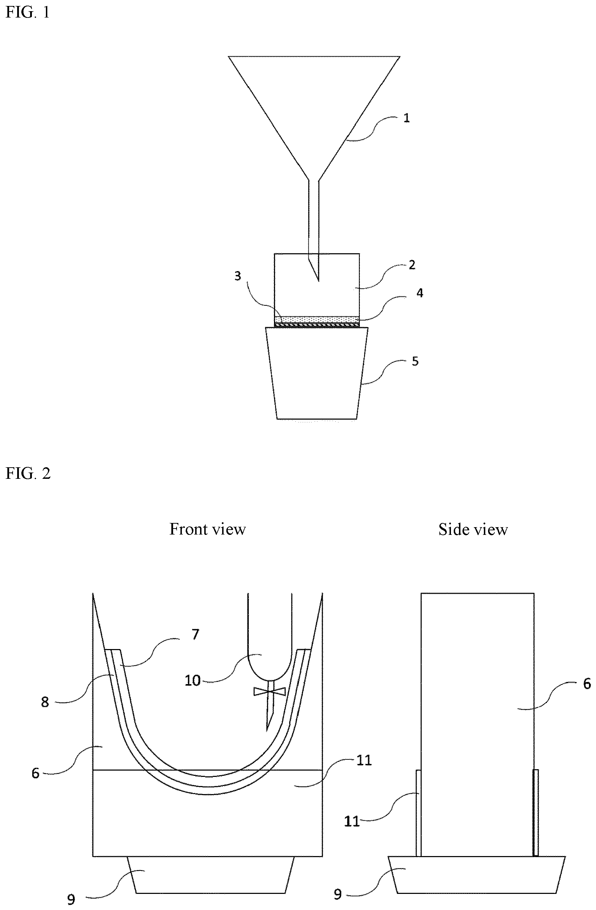

Washing of the water-absorbent resin composition was performed using the testing device shown in Fig. 1. In other words, a 200 mL plastic beaker with a height of 85 mm and an inner diameter of 74 mm is placed on a laboratory bench. A cylindrical container in which a stainless steel net having a mesh size of 36 $\mu$m is bonded to the bottom of a cylinder having an inner diameter of 60 mm × a height of 70 mm, is fixed above the cylindrical container using a clamp so that the bottom surface of the cylindrical container is located about 1 mm above the upper end of the beaker while maintaining a horizontal state. A plastic funnel having an inner diameter of 120 mm, a total length of 165 mm, a foot length of 70 mm, and a foot inner diameter of 9 mm is fixed by a clamp such that the center of the cylindrical container substantially coincides with the center of the funnel, and a distance between a tip of the funnel foot and the bottom surface of the cylindrical container is 55 mm. The measurement using the testing device was performed in the following procedure. The water-absorbent resin composition (1.00 g) was weighed and uniformly sprayed onto the cylindrical container, and then an instrument was fixed in an order of the beaker, the cylindrical container, and the funnel. Physiological saline of 200 g (0.9 mass% sodium chloride aqueous solution) was weighed and introduced into the funnel one time. In about 3 seconds, the whole amount of the physiological saline was dispersed in the cylindrical container. After about 1 minute, the washing liquid flowing out of the cylindrical container was acquired to obtain the first-washing washing liquid. Then, the beaker was exchanged and placed in a lower part of the device, and after 2 minutes from the start of the first introduction of the physiological saline, 200 g of physiological saline was further introduced to the funnel in the same manner as in the first time to obtain the second-washing washing liquid of the water-absorbent resin composition. The collected washing liquid (70 g) was weighed in a 100 mL plastic beaker, and a stirrer having a diameter of 8 mm and a length of 30 mm was put therein, and the mixture was stirred at 700 rpm. In a stirred state, an equilibrium value at a liquid temperature of 25 ± 1°C was read using a portable pH/ORP meter (D-72, electrode model 9625) manufactured by HORIBA, Ltd., and used as an electromotive force of the washing liquid. In addition, an equilibrium value is a value at a time when an electromotive force variation for consecutive 10 seconds becomes less than 1 mV at a time of electromotive force measurement.

<Median particle size of acidic compound>

An acidic compound (10 g) was sieved using a continuous fully automatic sonic vibration type sieving measuring instrument (Robot shifter RPS-205, manufactured by Seishin Enterprise Co., Ltd.), a sieve with JIS standard mesh sizes of 850 μm, 500 μm, 425 μm, 300 μm, 212 μm, 106 μm, 75 μm, and 45 μm, and a pan under sieving conditions of a frequency of 80 Hz, a pulse interval of 1 second, and a classification time of 2 minutes. A mass of the particles remaining on each sieve was calculated as a mass percentage with respect to a total amount. The mass percentage of the particles remaining on each sieve was integrated in a descending order of particle sizes, and the relationship between the mesh size and an integrated value of the mass percentage of particles remaining on the sieve was plotted on a logarithmic probability paper. By connecting the plots on the probability paper with a straight line, a particle size corresponding to an integrated mass percentage of 50 mass% was determined, and the particle size was defined as a median particle size.

<Median particle size of water-absorbent resin particles>

JIS standard sieves were combined in the following order from the top: a sieve with a mesh size of 850 μm, a sieve with a mesh size of 600 μm, a sieve with a mesh size of 500 μm, a sieve with a mesh size of 425 m, a sieve with a mesh size of 300 μm, a sieve with a mesh size of 250 μm, a sieve with a mesh size of 150 μm, and a receptacle. The water-absorbent resin composition (50 g) was placed on the uppermost sieve of the combined sieves, and shaken for 10 minutes using a Ro-Tap type (Rotating and Tapping type) shaker for classification. After classification, a mass of the water-absorbent resin particles remaining on each sieve was calculated as a mass percentage with respect to a total amount, and a particle size distribution was determined. With respect to the particle size distribution, a relationship between a mesh size of the sieve and an integrated value of the mass percentage of water-absorbent resin particles remaining on the sieve was plotted on a logarithmic probability paper by integrating the particles on the sieve in a descending order of particle size. By connecting the plots on the probability paper with a straight line, a particle size corresponding to an integrated mass percentage of 50 mass% was defined as a median particle size.

<Physiological saline absorption amount>

In a plastic beaker of 500 mL, 500 g of physiological saline and a stirrer (8 mmφ × 30 mm without a ring) were put, and stirred at 600 rpm using a magnetic stirrer. The water-absorbent resin composition (2.0 g) was dispersed in the beaker, and sufficiently swollen by gentle stirring at 600 rpm for 1 hour. On the other hand, the mass Wb (g) of a standard sieve with a mesh size of 75 μm was measured, and the aqueous solution containing a swollen gel was filtered through a standard sieve with a mesh size of 75 μm. A standard sieve of 75 μm was allowed to stand for 30 minutes in a state where the angle formed with respect to the horizontal was inclined at about 30 degrees, thereby removing excessive physiological saline from the water-absorbent resin composition. A sieve mass Wc (g) containing the swollen gel was measured, and a mass obtained by subtracting the mass Wb (g) of a 75 μm standard sieve from the mass Wc (g) was divided by the mass (2.0 g) of the water-absorbent resin composition to calculate the absorption amount.

$$\text{Absorption amount} = (Wc - Wb) \div (\text{water-absorbent resin composition mass})$$

<Preparation of absorbent article for evaluation>

Using an air flow mixer (Pad former manufactured by Autec Ltd.), 18.34 g of the water-absorbent resin composition and 12.48 g of pulverized pulp were uniformly mixed by air papermaking to prepare a sheet-shaped absorber having a size of 40 cm × 12 cm. The absorber was sandwiched between two pieces of tissue papers (core wraps) having the same size and a basis weight of 21 g/m$^2$ from above and below, and then the whole was pressed and molded at a load of 0.138 kPa for 30 seconds. The molded body was placed on a polyethylene sheet having the same size as the absorber and a basis weight of 40 g/m$^2$, and an air-through type porous liquid permeable sheet made of polyethylene-polypropylene having the same size as the absorber and a basis weight of 21 g/m$^2$ was further disposed on the upper surface of the molded body to obtain an absorbent article for evaluation having a layered configuration of a polyethylene sheet/tissue paper/absorber/tissue paper/air-through type porous liquid permeable sheet from the bottom.

<Method for producing artificial urine>

Sodium chloride (200 g), 6 g of calcium chloride dihydrate, 12 g of magnesium chloride hexahydrate, 50 g of a 1% Triton X-100 aqueous solution, and 1000 g of distilled water were put in a 1 L plastic beaker, and stirred and dissolved using a stirrer having a diameter of 8 mm and a length of 40 mm. The contents of the plastic beaker were introduced into a 20 L poly tank, the beaker was washed several times with distilled water, and the washing liquid was also merged into the poly tank. Furthermore, distilled water was added until the total mass of the solution in the poly tank reached 20 kg, and then 0.5 g of Blue No. 1 was added and stirred and dissolved to produce artificial urine.

<Liquid diffusion length of absorbent article>

The absorbent article for evaluation was placed on a horizontal table, and a cylindrical cylinder for liquid introduction having an inner diameter of 3 cm was placed at the center of the absorbent article for evaluation. Next, 100 mL of artificial urine adjusted to 25°C was put into the cylindrical cylinder one time. The absorption time from the start of introduction until the liquid was completely absorbed by the absorber was measured with a stopwatch. The cylinder was temporarily removed, and 10 minutes after the introduction of the liquid, the liquid diffusion length (D2) in the longitudinal direction starting from the liquid introducing position and the liquid diffusion lengths (D1 and D3) in the longitudinal direction starting from 1 cm inward from both right and left ends in the short direction of the absorbent article for evaluation were measured, and the average value of the diffusion lengths of D1 to D3 was obtained and rounded off to an integer value to obtain the liquid diffusion length.

<Liquid leakage (lateral leakage) test of absorbent article>

The liquid leakage performance test of the absorbent article for evaluation was performed using a testing device illustrated in Fig. 2. A U-shaped stand (6) (width 140 mm, depth 220 mm, height 215 mm, R = 83 R) was placed on an edge of a stainless steel square tray having a length of 168 mm, a width of 213 mm, and a height of 30 mm. The U-shaped stand is provided with a side wall (11, longest height 10 mm from U-shaped surface) for preventing the absorbent article for evaluation from slipping down from the U-shaped stand. In such a testing device, first, a position 8 cm above the center of the absorbent article for evaluation in the longitudinal direction was marked as a position to introduce the artificial urine. Next, the absorbent article for evaluation was placed so that the center of the absorbent article for evaluation coincided with the center of the U-shaped stand (6). A glass dropping funnel of 200 mL in which the squeezing of the tap was adjusted in advance so that the artificial urine passed at 8 mL/second was prepared, washed with the artificial urine, and then drained. Finally, the dropping funnel was fixed with a clamp so that the tip of the dropping funnel was 10 mm vertically above the artificial urine input position of the absorbent article for evaluation. As an evaluation operation, first, 80 mL of the artificial urine at 25°C was weighed into a 100 mL measuring cylinder and introduced into the dropping funnel one time, and the artificial urine was absorbed into the absorbent article for evaluation. The presence or absence of a liquid (liquid leakage) that flowed out onto the vat was confirmed 10 minutes after the introduction of the artificial urine, and when there was no liquid leakage, 80 mL of the artificial urine was introduced into the absorbent article for evaluation in the same manner as described above. The artificial urine was repeatedly introduced until liquid leakage was confirmed, and the number of times of introduction of the artificial urine until liquid leakage occurred was recorded.

<Production Example 1>

A round-bottomed cylindrical separable flask of 2 L having an inner diameter of 11 cm and equipped with a reflux condenser, a dropping funnel, a nitrogen gas introduction tube, and a stirring blade having two stages of 4-inclined paddle blades with a blade diameter of 5 cm was prepared as a stirrer. To this flask, n-heptane as a hydrocarbon dispersion medium of 293 g and maleic anhydride-modified ethylene-propylene copolymer of 0.736 g (Mitsui Chemicals, Inc., Hi-wax 1105 A) as a polymeric dispersant were added, and a temperature was raised to 80°C with stirring to dissolve the dispersant, then a mixture was cooled to 50°C. On the other hand, an 80.5 mass% aqueous solution of acrylic acid of 92.0 g (1.03 mol) as the water-soluble ethylenically unsaturated monomer was placed in a beaker with an internal volume of 300 mL, and a 20.9 mass% aqueous solution of sodium hydroxide of 147.7 g was added dropwise while cooling with ice water from an outside to perform neutralization at 75 mol%. Then, hydroxyl ethyl cellulose of 0.092 g (Sumitomo Seika Chemicals Company, Limited, HECAW -15 F) as a thickener, the potassium persulfate of 0.0736 g (0.272 mmol) as a water-soluble radical polymerization agent, and ethylene glycol diglycidyl ether of 0.010 g (0.057 mmol) as an internal crosslinking agent were added thereto and dissolved, thereby preparing a first-stage aqueous solution. Then, the aqueous solution prepared above was added to a separable flask and stirred for 10 minutes, and then a surfactant solution obtained by heating and dissolving sucrose stearate ester of 0.736 g (Ryoto sugar ester S -370 manufactured by Mitsubishi Chemical Foods Corporation) with HLB3 as a surfactant in n-heptane of 6.62 g was further added. While a rotation speed of a stirrer was set to 550 rpm and stirring, an inside of the system was sufficiently replaced with nitrogen. Thereafter, the flask was immersed in a water bath at 70°C and heated, and polymerization was performed for 60 minutes to obtain a first-stage polymerization slurry solution. On the other hand, an 80.5 mass% aqueous solution of acrylic acid of 128.8 g (1.43 mol) as the water-soluble ethylenically unsaturated monomer was placed in another beaker with an internal volume of 500 mL, a 27 mass% aqueous solution of sodium hydroxide of 159.0 g was added dropwise with external cooling to perform neutralization at 75 mol%, and then the potassium persulfate of 0.103 g (0.381 mmol) as a water-soluble radical polymerization initiator and ethylene glycol diglycidyl ether of 0.0116 g (0.067 mmol) as an internal crosslinking agent were added and dissolved, thereby preparing a second-stage aqueous liquid. An inside of a separable flask system was cooled to 25°C while a rotation speed of the stirrer was set to 1000 rpm, and then a whole amount of a second-stage aqueous liquid was added to the first-stage polymerization slurry, and the inside of the system was replaced with nitrogen for 30 minutes. Then, the flask was immersed in a water bath at 70°C again, the temperature was raised, and the polymerization reaction was performed for 60 minutes to obtain a hydrous gel polymer. A 45 mass% aqueous pentasodium diethylenetriamine pentaacetate solution (0.589 g) was added to a hydrous gel polymer after a second-stage polymerization under stirring. Thereafter, the flask was immersed in an oil bath set at 125°C, and water of 257.7 g was removed to the outside of the system while refluxing n-heptane by azeotropic distillation of n-heptane and water. Thereafter, a 2 mass% aqueous solution of ethylene glycol diglycidyl ether of 4.42 g (0.507 mmol) as a surface crosslinking agent was added to the flask, and the mixture was held at 83°C for 2 hours. Thereafter, n-heptane was evaporated at 125°C and dried to obtain polymer particles (dry product). The polymer particles were passed through a sieve with a mesh size of 850 $\mu$m to obtain 228.0 g of water-absorbent resin particles. The median particle size of the water-absorbent resin particles was 394 $\mu$m.

<Example 1>

Powder of 0.1 parts by mass of L-tartaric acid (Product name: purified L-tartaric acid, the first acid dissociation constant pKa1 = 2.87, the second acid dissociation constant pKa2 = 3.97, manufactured by FUSO CHEMICAL INDUSTRY CO., LTD., median particle size 280 $\mu$m) was mixed with 100 parts by mass of the water-absorbent resin particles obtained in Production Example 1 to obtain a water-absorbent resin composition. Various performance measurements of the obtained water-absorbent resin composition and an evaluation of an absorbent article using the same were performed. The results are shown in Table 1.

<Example 2>

A water-absorbent resin composition was obtained in the same manner as in Example 1 except that L-tartaric acid was changed to 0.5 parts by mass based on 100 parts by mass of the water-absorbent resin particles in Example 1. Various performance measurements of the obtained water-absorbent resin composition and an evaluation of an absorbent article using the same were performed. The results are shown in Table 1.

<Example 3>

A water-absorbent resin composition was obtained in the same manner as in Example 1 except that L-tartaric acid was changed to 1.0 parts by mass relative to 100 parts by mass of the water-absorbent resin particles in Example 1. Various performance measurements of the obtained water-absorbent resin composition and an evaluation of an absorbent article using the same were performed. The results are shown in Table 1.

<Example 4>

A water-absorbent resin composition was obtained in the same manner as in Example 1 except that L-tartaric acid was changed to 2.0 parts by mass relative to 100 parts by mass of the water-absorbent resin particles in Example 1. Various performance measurements of the obtained water-absorbent resin composition and an evaluation of an absorbent article using the same were performed. The results are shown in Table 1.

<Example 5>

A water-absorbent resin composition was obtained in the same manner as in Example 1 except that L-tartaric acid was changed to 3.0 parts by mass relative to 100 parts by mass of the water-absorbent resin particles in Example 1. Various performance measurements of the obtained water-absorbent resin composition and an evaluation of an absorbent article using the same were performed. The results are shown in Table 1.

<Example 6>

A water-absorbent resin composition was obtained in the same manner as in Example 1 except that L-tartaric acid was changed to 5.0 parts by mass relative to 100 parts by mass of the water-absorbent resin particles in Example 1. Various performance measurements of the obtained water-absorbent resin composition and an evaluation of an absorbent article using the same were performed. The results are shown in Table 1.

<Example 7>

A water-absorbent resin composition was obtained in the same manner as in Example 1 except that L-tartaric acid was changed to 10.0 parts by mass relative to 100 parts by mass of the water-absorbent resin particles in Example 1. Various performance measurements of the obtained water-absorbent resin composition and an evaluation of an absorbent article using the same were performed. The results are shown in Table 1.

<Example 8>

Powder of 1.0 parts by mass of citric acid (manufactured by FUSO CHEMICAL INDUSTRY CO., LTD., product name: Fuso citrate (anhydrous), the first acid dissociation constant $pKa1 = 2.90$, the second acid dissociation constant $pKa2 = 4.35$, the third acid dissociation constant $pKa3 = 5.69$, median particle size 236 $\mu$m) was mixed with 100 parts by mass of the water-absorbent resin particles obtained in Production Example 1 to obtain a water-absorbent resin composition. Various performance measurements of the obtained water-absorbent resin composition and an evaluation of an absorbent article using the same were performed. The results are shown in Table 1.

<Example 9>

Powder of 1.0 parts by mass of DL-malic acid (manufactured by FUSO CHEMICAL INDUSTRY CO., LTD., product name: malic acid FUSO, the first acid dissociation constant $pKa1 = 3.23$, the second acid dissociation constant $pKa2 = 4.77$, median particle size 156 $\mu$m) was mixed with 100 parts by mass of the water-absorbent resin particles obtained in Production Example 1 to obtain a water-absorbent resin composition. Various performance measurements of the obtained water-absorbent resin composition and an evaluation of an absorbent article using the same were performed. The results are shown in Table 1.

<Example 10>

Powder of 1.0 parts by mass of fumaric acid (manufactured by FUSO CHEMICAL INDUSTRY CO., LTD., product name: fumaric acid, the first acid dissociation constant $pKa1 = 3.07$, the second acid dissociation constant $pKa2 = 4.58$, median particle size 161 $\mu$m) was mixed with 100 parts by mass of the water-absorbent resin particles obtained in Production Example 1 to obtain a water-absorbent resin composition. Various performance measurements of the obtained water-absorbent resin composition and an evaluation of an absorbent article using the same were performed. The results are shown in Table 1.

<Comparative Example 1>

The water-absorbent resin particles obtained in Production Example 1 were used as they were as water-absorbent resin particles of Comparative Example 1. Various performance measurements of water-absorbent resin particles and an evaluation of an absorbent article using the same were performed. The results are shown in Table 1.

[Table 1]

| | Water-absorbent resin composition | | Various performance of water-absorbent resin composition | | | | Evaluation of absorbent article | |
|---|---|---|---|---|---|---|---|---|
| | Acidic compound | Addition amount (parts by mass) | Electromotive force (mV) of washing lic uid | | | Physiological saline absorption amount (g/g) | Diffusion length (cm) | Number of times until liquid leakage |
| | | | First (P) | Second (Q) | Difference (P-Q) | | | |
| Example 1 | L-tartaric acid | 0.1 | 92 | 56 | 36 | 61 | 19 | 6 |
| Example 2 | L-tartaric acid | 0.5 | 133 | 59 | 74 | 61 | 19 | 6 |
| Example 3 | L-tartaric acid | 1 | 143 | 64 | 79 | 60 | 20 | 6 |
| Example 4 | L-tartaric acid | 2 | 167 | 61 | 106 | 60 | 21 | 6 |
| Example 5 | L-tartaric acid | 3 | 169 | 84 | 85 | 58 | 21 | 6 |
| Example 6 | L-tartaric acid | 5 | 175 | 76 | 99 | 57 | 21 | 6 |
| Example 7 | L-tartaric acid | 10 | 194 | 96 | 98 | 55 | 22 | 5 |
| Example 8 | Citric acid | 1 | 147 | 59 | 88 | 61 | 21 | 6 |
| Example 9 | DL-malic acid | 1 | 138 | 61 | 77 | 61 | 19 | 5 |
| Example 10 | Fumaric acid | 1 | 107 | 74 | 33 | 60 | 20 | 5 |
| Comparative Example 1 | - | | 61 | 58 | 3 | 61 | 18 | 5 |

DESCRIPTION OF REFERENCE SIGNS

1: Funnel

2: Cylindrical container

3: Sieve mesh made of stainless steel having a mesh size of 36 $\mu$m

4: Water-absorbent resin composition

5: Plastic beaker of 200 mL

6: U-shaped stand

7: Absorbent article for evaluation

8: Polyethylene sheet

9: Stainless steel square tray

10: Dropping funnel

11: Side wall

**Claims**

1. A water-absorbent resin composition, wherein

   an electromotive force P (mV) of a first-washing washing liquid obtained by washing the water-absorbent resin composition by the following washing method is 80 mV or more, and
   a difference (P - Q) between an electromotive force Q (mV) of a second-washing washing liquid obtained by further washing the water-absorbent resin composition by the following washing method and the electromotive force P (mV) is 10 mV or more, wherein
   (washing method)
   a container in which a sieve mesh having a mesh size of 36 $\mu$m is attached to a bottom of a cylinder having an inner diameter of 60 mm is prepared; the water-absorbent resin composition of 1 g is uniformly sprayed onto the sieve mesh in the container; a beaker of 200 mL is placed under the sieve mesh; physiological saline of 200 g is quickly introduced from above the water-absorbent resin composition, and the physiological saline flowing down into the beaker is used as the first-washing washing liquid; next, a beaker of 200 mL is newly placed under the sieve mesh; physiological saline of 200 g is further quickly introduced from above the water-absorbent resin composition, and the physiological saline flowing down into the beaker is used as the second-washing washing liquid.

2. The water-absorbent resin composition according to claim 1, wherein the electromotive force Q (mV) of the second-washing washing liquid is 130 mV or less.

3. The water-absorbent resin composition according to claim 1 or 2, wherein the water-absorbent resin composition has a physiological saline absorption amount of 30 to 80 g/g.

4. The water-absorbent resin composition according to any one of claims 1 to 3, comprising water-absorbent resin particles and an acidic compound.

5. The water-absorbent resin composition according to claim 4, wherein the water-absorbent resin particles have a median particle size of 200 to 600 $\mu$m, and the acidic compound has a median particle size of 20 to 600 $\mu$m.

6. The water-absorbent resin composition according to claim 4 or 5, wherein the acidic compound is contained in an amount of 0.05 to 30 parts by mass with respect to 100 parts by mass of the water-absorbent resin particles.

7. The water-absorbent resin composition according to any one of claims 4 to 6, wherein a ratio (T/S) of a median particle size T ($\mu$m) of the water-absorbent resin particles to a median particle size S ($\mu$m) of the acidic compound is 0.1 to 30.

8. The water-absorbent resin composition according to any one of claims 4 to 7, wherein a first acid dissociation constant of the acidic compound is 0.1 to 5.0.

9. An absorber comprising the water-absorbent resin composition according to any one of claims 1 to 8.

10. An absorbent article comprising the absorber according to claim 9.

FIG. 1

FIG. 2

Front view                           Side view

## INTERNATIONAL SEARCH REPORT

| International application No. |
|---|
| PCT/JP2021/026700 |

A.  CLASSIFICATION OF SUBJECT MATTER
Int.Cl.  C08F20/06(2006.01)i, A61F13/53(2006.01)i, B01J20/26(2006.01)i,
B01J20/28(2006.01)i
FI: B01J20/26D, A61F13/53300, B01J20/28Z, C08F20/06
According to International Patent Classification (IPC) or to both national classification and IPC

B.  FIELDS SEARCHED

Minimum documentation searched (classification system followed by classification symbols)
Int.Cl.  C08F20/06, A61F13/53, B01J20/26, B01J20/28

Documentation searched other than minimum documentation to the extent that such documents are included in the fields searched
Published examined utility model applications of Japan        1922-1996
Published unexamined utility model applications of Japan      1971-2021
Registered utility model specifications of Japan              1996-2021
Published registered utility model applications of Japan      1994-2021

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| X | JP 2005-186016 A (SAN-DIA POLYMER LTD.) 14 July 2005 (2005-07-14), claims 1-5, paragraphs [0045], [0058], [0059], [0073], examples 1-15, 17, table 1 | 1-10 |
| X | WO 2014/054656 A1 (NIPPON SHOKUBAI CO., LTD.) 10 April 2014 (2014-04-10), claims 11, 14, paragraph [0078], examples 2-20 | 1-10 |
| A | JP 2017-110091 A (EARTH CHEMICAL CO., LTD.) 22 June 2017 (2017-06-22), entire text | 1-10 |

☐  Further documents are listed in the continuation of Box C.      ☒  See patent family annex.

| | |
|---|---|
| *    Special categories of cited documents: | "T"  later document published after the international filing date or priority date and not in conflict with the application but cited to understand the principle or theory underlying the invention |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
|---|---|
| 06 August 2021 | 31 August 2021 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**EP 4 186 930 A1**

| INTERNATIONAL SEARCH REPORT | International application No. |
|---|---|
| Information on patent family members | PCT/JP2021/026700 |

```
JP 2005-186016 A   14 July 2005      (Family: none)

WO 2014/054656 A1  10 April 2014     US 2015/0258237 A1
                                     claims 11, 14, paragraph [0085],
                                     examples 2-20
                                     EP 2905071 A1
                                     CN 104703690 A
                                     KR 10-2015-0064051 A

JP 2017-110091 A   22 June 2017      (Family: none)
```

Form PCT/ISA/210 (patent family annex) (January 2015)

**EP 4 186 930 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H3227301 A